# NEUE EUROPÄISCHE PATENTSCHRIFT

(11) **EP 2 473 090 B2**
(45) Veröffentlichungstag und Bekanntmachung des Hinweises auf die Entscheidung über den Einspruch: **29.11.2017**
(45) Hinweis auf die Patenterteilung: 12.02.2014
(21) Anmeldenummer: 10745146.0
(22) Anmeldetag: 05.08.2010
(51) Int. Cl.: A61B 1/07, A61B 5/00

(54) **MEDIZINISCHE LEUCHTE FÜR HINTERGRUNDLICHT UND ANREGUNGSLICHT**
MEDICAL LUMINAIRE FOR BACKGROUND LIGHT AND EXCITATION LIGHT
APPAREIL D'ÉCLAIRAGE MÉDICAL POUR LUMIÈRE D'ARRIÈRE-PLAN ET LUMIÈRE D'EXCITATION

(30) Priorität: 04.09.2009 DE 102009040093
(43) Veröffentlichungstag der Anmeldung: 11.07.2012
(73) Patentinhaber: Olympus Winter&Ibe GmbH, 22045 Hamburg (DE)
(72) Erfinder: WOLTER, Michael, 22047 Hamburg (DE)
(74) Vertreter: Hausfeld, Norbert
(86) Internationale Anmeldenummer: PCT/EP2010/004798
(87) Internationale Veröffentlichungsnummer: WO 2011/026548

(56) Entgegenhaltungen:
- EP-A1- 0 512 965
- DE-A1- 10 002 106
- DE-A1- 10 031 530
- JP-A- 2005 218 760
- US-A1- 2001 049 473
- US-A1- 2004 186 351
- US-A1- 2006 184 037

## Beschreibung

Die Erfindung betrifft eine medizinische Leuchte der im Oberbegriff des Anspruches 1 genannten Art.

Photo-Dynamische Diagnose (PDD) wird hauptsächlich zur Erkennung von Tumoren verwendet, die z. B. nach Verabreichung bestimmter Substanzen fluorezieren. Dazu werden Leuchten benötigt, die kurzwelliges Anregungslicht auf das zu beobachtende Gebiet abgeben, durch welches die fluoreszierenden Bereiche zur Fluoreszenz im langwelligen Bereich angeregt werden. Die Beobachtung erfolgt zumeist durch einen Langpassfilter zur Unterdrückung des kurzwelligen Anregungslichtes. Einzelheiten zu diesem Stand der Technik lassen sich z. B. in DE 19902184 C1 oder DE 19639653 A1 nachlesen.

Eine gattungsgemäße Leuchte ist in der DE 102006011749 A1 beschrieben. Als Breitbandlampe dient hier eine Anordnung aus mehreren Leuchtdioden, die gemeinsam weißes Licht erzeugen und von denen eine, die blaues Licht erzeugt, als die Halbleiterlampe zur Erzeugung des Anregungslichtes dient. Die Halbleiterlampe dient also sowohl zur Erzeugung des Anregungslichtes als auch zur Erzeugung des entsprechenden Spektralbereiches des Breitbandlichtes.

Daraus resultieren allerdings auch Nachteile, vor allem weil die Halbleiterlampe bei dieser Konstruktionsart relativ breitbandig sein muss. Sie erzeugt also ein sehr intensives, alles überstrahlendes Blau, von dem nur ein geringer, zur Anregung nutzbarer Frequenzbereich tatsächlich die Fluoreszenz anregt. Das intensive Blau muss mit einem sehr wirkungsvollen Langpassfilter ausgefiltert werden, um bei der Beobachtung nicht das gesamte Bild, einschließlich der Fluoreszenzeffekte zu überstrahlen.

Aus der DE 101 36 191 A1 und der DE 93 17 984 U1 sind gattungsgemäße Leuchten bekannt, bei denen sowohl das Licht der Breitbandlampe als auch das Anregungslicht gemeinsam in einem Lichtleitfaserbündel transportiert werden. Als Halbleiterlampe wird eine Laserdiode verwendet. Es ergibt sich also eine sehr einfache Konstruktion.

Nachteilig bei diesen Konstruktionen ist dagegen, dass sowohl das langwellige Licht als auch das kurze Anregungslicht durch dieselben Glasfasern, also durch dasselbe Glas verlaufen. Das führt zu Nachteilen, da die Glassorten nicht optimal an die Wellenlängen angepasst sind.

Die US2001049473 A zeigt in Fig. 6 eine Konstruktion, bei der beide Lichtarten durch denselben Lichtleiter verlaufen. In Fig. 12 ist eine Konstruktion gezeigt, bei der für jede Lichtart ein eigener Lichtleiter vorgesehen ist. Diese beiden Lichtleiter verlaufen zunächst getrennt und werden dann zu einem Kabel verbündelt. Die US2004186351 A lässt die beiden Lichtarten durch getrennte Lichtleiter laufen oder durch einen gemeinsamen Lichtleiter. Die DE10031530 A sieht getrennte Lichtleiter vor. Die EP0512965 A schickt beide Lichtarten durch einen gemeinsamen Lichtleiter. Die JP2005218760 A sieht wiederum für die beiden Lichtarten getrennte Lichtleiter vor.

In allen diesen fünf letztgenannten Schriften gibt es nur die Alternative, entweder die beiden Lichtarten durch denselben Lichtleiter zu schicken, oder durch zwei getrennte Lichtleiter. Letzteres hat insbesondere den Nachteil, dass zwei Eingangskupplungen für Lichtleiter benötigt werden.

Die Aufgabe der vorliegenden Erfindung besteht darin, eine einfache und kostengünstige Lösung dieses Beleuchtungsproblems zu schaffen.

Diese Aufgabe wird mit den Merkmalen des Kennzeichnungsteils des Anspruches 1 gelöst.

Erfindungsgemäß wird das Anregungslicht in einem gesonderten Teilbündel transportiert. Dieses kann daher auf die Wellenlänge des Anregungslichtes optimiert sein, z. B. aus Quarzglas bestehen, während das übrige Teilbündel in der üblichen Weise für weißes Licht optimiert ist.

Eine überlagernde Einspeisuhg des Anregungslichtes zum Breitbandlicht ist auf vielen Wegen möglich, vorteilhaft jedoch gemäß Anspruch 2 mit einem Spiegel, der z. B. aufgrund des Einstrahlwinkels oder der unterschiedlichen Lichtfrequenz von dem einen Licht durchsetzt wird, während das andere daran reflektiert wird.

Diese Auswahleigenschaft des Spiegels hinsichtlich des einen oder anderen Lichtes kann wiederum auf unterschiedliche Weise bewirkt werden, vorteilhaft jedoch gemäß Anspruch 3, indem das durchsetzende Licht den Spiegel in einem Loch durchsetzt. Dies ist eine konstruktiv besonders einfache Möglichkeit.

In der Zeichnung ist die Erfindung beispielsweise und schematisch dargestellt. Es zeigen:
- Fig. 1: eine schematische Ansicht eines Endoskops mit erfindungsgemäßer Leuchte,
- Fig. 2: einen vergrößerten Schnitt nach Linie 2 - 2 in Fig. 1 und
- Fig. 3: einen Schnitt durch die Leuchte der Fig. 1.

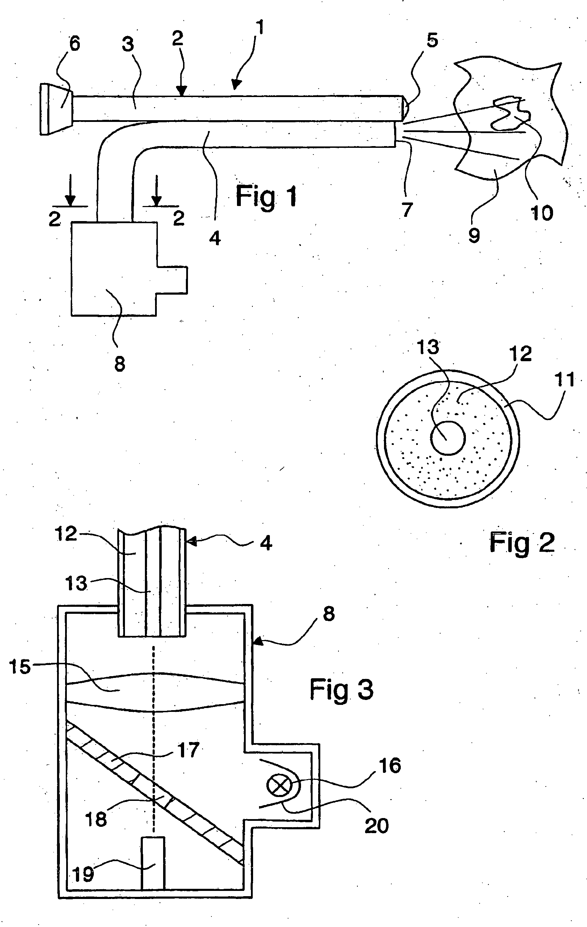

Fig. 1 zeigt ein Endoskop 1, in dessen Schaftbereich 2 ein Bildleiter 3 und ein Faserlichtleiter 4 parallel angeordnet sind. Ein üblicherweise vorhandenes, den Bildleiter 3 und den Faserlichtleiter 4 umschließendes Außenrohr, das auch noch zusätzliche Kanäle enthalten kann, ist der zeichnerischen Vereinfachung wegen weggelassen.

Der Bildleiter 3, der beispielsweise als Bildleitfaserbündel oder auch als Relaislinsenanordnung ausgebildet sein kann, weist an seinem distalen Ende ein Objektiv 5 auf und an seinem proximalen Ende ein Okular 6. Statt des Okulars 6 kann auch eine Kamera vorgesehen sein, die auch distal im Bildleiter 3 angeordnet sein kann.

Der Faserlichtleiter 4 besteht aus einem Lichtleitfaserbündel, das von seiner distalen Stirnfläche 7 Licht abstrahlt und an seinem proximalen Ende mit einer Leuchte 8 mit Licht versorgt wird. Der Faserbildleiter 4 und/oder der Bildleiter 3 können flexibel ausgebildet sein.

Der Schaftbereich 2 des Endoskops 1 ist auf eine Gewebeoberfläche 9 gerichtet, die in Fig. 1 als Ausschnitt dargestellt ist. Auf dem dargestellten Ausschnitt der Gewebeoberfläche 9 liegt ein Tumor 10, der vom Licht des Faserlichtleiters 4 bestrahlt und vom Objektiv 5 des Bildleiters 3 betrachtet wird.

Im erfindungsgemäßen Ausführungsbespiel nach Fig. 2 ist der Faserlichtleiter 4, auf besondere Weise zum Transport unterschiedlicher Lichtarten ausgerüstet. In einer Schutzumhüllung 11 liegt in üblicher Weise das Lichtleitfaserbündel 12, das den Faserlichtleiter 4 bildet. Im Lichtleitfaserbündel 12 ist ein gesondertes Teilbündel 13 ausgebildet, das im Ausführungsbeispiel zentral angeordnet ist.

Das Teilbündel 13 kann wiederum als Bündel von Lichtleitfasern ausgebildet sein, oder auch aus einer einzelnen Faser bestehen. Es ist besonders für kurzwelliges Licht optimiert, kann also z. B. aus Quarz bestehen, während das restliche Bündel 12 aus Glas besteht.

Das Innere der Leuchte 8 ist in Fig. 3 in einem Schnitt dargestellt. In einer Durchbrechung eines Gehäuses 14 ist das proximale Ende des Faserlichtleiters 4 befestigt. Durch einen Kondensor 15, der schematisch als Linse dargestellt ist, wird die proximale Stirnfläche des Faserlichtleiters 4 von einer Breitbandlampe 16 über einen unter 45° zur Achse des proximalen Endbereiches des Faserlichtleiters 4 angeordneten Spiegel 17 beleuchtet. Der Spiegel 17 weist in seiner Mitte ein Loch 18 auf, durch das eine Laserdiode 19 ein schmales Lichtbündel unmittelbar auf den Bereich des Teilbündels 13 richtet.

Die Laserdiode 19 ist für kurzwelliges Licht im blauen, bzw. ultravioletten Spektrum ausgebildet und in ihrem schmalbandigen Abgabespektrum passend zu dem Absorbtionsspektrum einer Fluoreszenzsubstanz ausgesucht, die dem Gewebe 9 der Fig. 1 zugegeben ist, um den Tumor 10 fluoreszieren zu lassen.

Bei der Anordnung der Fig. 3 wird das von der Laserdiode 19 erzeugte Licht in dem Teilbündel 13 getrennt von dem Licht der Lampe 16 transportiert, das im übrigen Querschnittsbereich des Faserlichtleiters 4 transportiert wird.

In einer nicht erfindungsgemäßen Abwandlung dieser Konstruktion kann der Faserlichtleiter 4 auch durchgehend aus Fasern derselben Sorte ausgebildet sein, die beide Lichtarten, also das Licht der Lampe 16 und das Licht der Laserdiode 19 gemeinsam transportieren.

Auch kann die Überlagerung der beiden Lichtarten von der Lampe 16 und der Laserdiode 19 auf andere Weise als dargestellt erreicht werden, z. B. indem der Spiegel 18 ohne Loch ausgebildet ist, aber z. B. für das Licht der Laserdiode 19 durchlässig ist, während er das Licht der Lampe 16 reflektiert.

Die Lampe 16 ist mit einem Reflektor 20 dargestellt. Sie kann eine konventionelle Breitbandlampe, z. B. eine Xenon-Lampe sein oder auch als Halbleiterlampe, z. B. aus mehreren Leuchtdioden bestehen, die gemeinsam breitbandiges, z. B. weißes Licht erzeugen.

Anstelle, wie dargestellt, das Licht der Laserdiode 19 dem der Breitbandlampe 16 zu überlagern, kann auch mit abwechselndem Licht gearbeitet werden. Anstelle des Spiegels 17 kann z. B. ein bewegter Spiegel vorgesehen sein, der abwechselnd das eine und das andere Licht in Richtung auf den Faserbildleiter 4 passieren lässt.

Bei Verwendung einer planaren Breitbandlampe, die z.B. als flacher Chip mit darauf angeordneten LEDs ausgebildet ist, kann auf den Spiegel 17 verzichtet werden und das Licht der Laserdiode 19 ein Loch im Chip der Breitbandlampe durchlaufen.

## Patentansprüche

1. Medizinische Leuchte (8) für die Photo-Dynamische Diagnose mit einer Lampe (16) zur Erzeugung von breitbandigen Hintergrundlicht sowie mit einer Halbleiterlampe (19) zur Erzeugung von kurzweiligem Anregungslicht, wobei die Halbleitertampe als Laserdiode (19) ausgebildet ist, deren Licht alternativ oder zusätzlich in den Lichtweg der Breitbandlampe (16) gespeist werden kann, wobei ein Lichtleitfaserbündel (12) zum Transport des Lichtes vorgesehen ist, wobei die Laserdiode derart angeordnet und ausgebildet ist, dass das Licht der Laserdiode (19) vor dem Eintritt in das Lichtleitfaserbündel (12) in den Lichtweg der Breitbandlampe (16) gespeist werden kann, **dadurch gekennzeichnet, dass** das Anregungslicht in einem gesonderten Teilbündel (13) transportiert werden kann, das im Lichtleitfaserbündel (12) ausgebildet ist, und wobei das Teilbündel auf die Wellenlänge des Anregungslichtes optimiert ist während das übrige Teilbündel in der üblichen Weise für weißes Licht optimiert ist.

2. Leuchte nach Anspruch 1, **dadurch gekennzeichnet, dass** das Anregungslicht in das Hintergrundlicht mit einem Spiegel (17) eingespeist wird, der von dem einen Licht durchsetzt wird, während er das andere Licht reflektiert.

3. Leuchte nach Anspruch 2, **dadurch gekennzeichnet, dass** der Spiegel (17) ein das Anregungslicht durchlassendes Loch (18) aufweist.

## Claims

1. A medical luminaire (8) for the photo-dynamic diagnosis, comprising a lamp (16) for generating broad-band background light as well as comprising a semiconductor lamp (19) for generating short-wave excitation light, wherein the semiconductor lamp is embodied as laser diode (19), the light of which can be fed, either as an alternative or in addition, into the optical path of the broad-band lamp (16), wherein provision is made for an optical fibre bundle (12) for transporting the light, wherein the laser diode is arranged and embodied such that the light from the laser diode (19) can be fed into the optical path of the broad-band lamp (16) prior to entering the optical fibre bundle (12), **characterised in that** the excitation light can be transported in a separate partial bundle (13), which is embodied in the optical fibre bundle (12), and wherein the partial bundle is optimized for the wavelength of the excitation light, while the remaining partial bundle is optimized for white light in the usual way.

2. The luminaire according to claim 1, **characterised in that** the excitation light is fed into the background light by mean of a mirror (17), which is permeated by the one light, while it reflects the other light.

3. The luminaire according to claim 2, **characterised in that** the mirror (17) includes a hole (18), which allows the excitation light to pass.

## Revendications

1. Appareil d'éclairage médical (8) pour diagnostic photo-dynamique avec une lampe (16) pour la production d'une lumière d'arrière-plan à large bande ainsi qu'une lampe à semi-conducteur (19) pour la production d'une lumière d'excitation de courte longueur d'onde, la lampe à semi-conducteur étant réalisée sous forme de diode laser (19) dont la lumière peut, en alternative ou additionnellement, être dirigée dans le trajet du faisceau lumineux de la lampe à large bande (16), un faisceau de fibres optiques (12) étant prévu pour le transport de la lumière et la diode laser étant réalisée et agencée de façon telle que la lumière de la diode laser (19) peut être dirigée dans le trajet du faisceau lumineux de la lampe à large bande (16) avant d'entrer dans le faisceau de fibres optiques (12), **caractérisé en ce que** la lumière d'excitation peut être transportée dans un faisceau partiel (13) séparé formé dans le faisceau de fibres optiques (12), le faisceau partiel étant optimisé pour la longueur d'onde de la lumière d'excitation alors que le faisceau partiel restant est, de manière usuelle, optimisé pour la lumière blanche.

2. Appareil d'éclairage selon la revendication 1, **caractérisé en ce que** la lumière d'excitation est dirigée dans la lumière d'arrière-plan par un miroir (17) traversé par l'une des lumières et réfléchissant l'autre lumière.

3. Appareil d'éclairage selon la revendication 2, **caractérisé en ce que** le miroir (17) présente un trou (18) laissant passer la lumière d'excitation.
